# DEMANDE DE BREVET EUROPEEN

(11) **EP 1 396 258 A1**
(43) Date de publication de la demande: **10.03.2004**
(21) Numéro de dépôt: 03102698.2
(22) Date de dépôt: 04.09.2003
(51) Int. Cl.: A61K 7/032, A61K 7/06

(54) **Composition cosmetique non rincée de maquillage ou de soin des fibres keratiniques sans cire**

(30) Priorité: 06.09.2002 FR 0211091
(71) Demandeur: L'OREAL, 75008 Paris (FR)
(72) Inventeur: DE LA POTERIE, Valérie, 77820, LE CHATELET EN BRIE (FR); LION, Bertrand, 95270, LUZARCHES (FR)
(74) Mandataire: Poulin, Gérard

(57) **Abrégé**

Composition cosmétique non rincée de maquillage ou de soin des fibres kératiniques, comprenant au moins une phase grasse comprenant au moins un agent structurant constitué par l'association d'un composé particulier et d'au moins une huile, ladite composition ne contenant pas de cires et présentant une teneur en solides définie par un extrait sec en solides supérieur à 45 % en poids.

## Description

La présente invention concerne une composition cosmétique non rincée de maquillage ou de soin des fibres kératiniques ne contenant pas de cires.

L'invention a trait également à l'utilisation de cette composition pour le maquillage des fibres kératiniques, notamment des cils, sourcils et cheveux, ainsi qu'à un procédé de maquillage ou de soin cosmétique de ces dernières.

La composition et le procédé de maquillage, selon l'invention, sont plus particulièrement destinés aux fibres kératiniques, notamment sensiblement longitudinales, d'êtres humains, telles que les cils, les sourcils et les cheveux, y compris les faux cils, de préférence la composition et le procédé de maquillage, selon l'invention sont destinés aux cils.

La composition peut être une composition de maquillage, une base de maquillage, dite encore « base coat », une composition à appliquer sur un maquillage, dite encore « top-coat », ou bien encore une composition de traitement cosmétique, de soin, des fibres kératiniques. Plus particulièrement, l'invention porte sur une composition que l'on peut définir comme une composition de maquillage des yeux, telle qu'un mascara.

Les compositions de maquillage des yeux et en particulier des cils, telles que les mascaras, peuvent se présenter sous différentes formes : par exemple, sous la forme d'émulsions diphasiques huile-dans-eau ou H/E ou eau-dans-huile E/H, de dispersions aqueuses ou anhydres.

Ces compositions sont caractérisées par leur teneur en solides qui est apportée en partie par une phase grasse dispersée constituée, par exemple, d'une ou plusieurs cires afin d'apporter de la matière sur les cils et donc un résultat maquillage volumateur.

Il est connu de l'art antérieur que plus la teneur en solides dans une composition va augmenter plus le dépôt de matière sur le cil va être important et donc plus le résultat obtenu sera volumateur.

Néanmoins, l'augmentation de la teneur en solides, c'est-à-dire le plus souvent de la quantité de cires dans une composition, telle qu'une émulsion ou dispersion entraîne une augmentation de la consistance du produit obtenu et donc une application sur les cils délicate et difficile car le produit est épais, visqueux, il se dépose difficilement, de façon hétérogène et par paquets.

L'augmentation de la teneur en solides est donc souvent limité par l'augmentation de la consistance et ne dépasse pas 45% du poids total de la composition.

Cette limitation sur la teneur en solides est souvent liée à l'impossibilité d'augmenter la teneur en cires dans la phase grasse qui ne dépasse pas 25% pour des raisons de faisabilité ; et entre 20% et 25% en poids de cire les compositions sont souvent très épaisses, compactes, difficiles à appliquer et présentent des propriétés cosmétiques non satisfaisantes.

C'est généralement le cas des mascaras dits volumateurs qui sont difficiles à appliquer et donnent un maquillage hétérogène.

Un autre moyen d'augmenter la teneur en solides est d'incorporer des particules solides comme des charges ou des pigments mais l'augmentation de la consistance limite également le pourcentage maximum en solides, de plus l'utilisation de particules solides en quantité importante ne favorise pas le dépôt homogène et lisse en raison non seulement de la consistance mais aussi de la taille des particules introduites qui donne un aspect granuleux et non lisse au dépôt.

A l'opposé, il est possible de formuler des compositions de consistance faible et faciles à appliquer mais la teneur en cires est alors faible entraînant un effet maquillant non satisfaisant.

Il a également déjà été décrit des compositions de maquillage des yeux sans cires. Celles-ci sont généralement basées sur l'utilisation de polymères filmogènes, mais il n'est pas possible de les formuler à de fortes teneurs en solides car soit la consistance augmente fortement dans le cas des polymères solubles soit la teneur en solides est limitée car ils se présentent sous la forme d'émulsions ou de dispersions aqueuses ou anhydres.

Il n'est donc pas possible d'obtenir une composition de maquillage des fibres kératiniques, en particulier une composition dite de « maquillage des yeux » exempte de cires qui comprennent une forte teneur en solides. Un autre avantage est de pouvoir obtenir des compositions qui présentent, de préférence, une consistance faible pour une application facile et homogène alliée à un effet volumateur et séparant satisfaisant.

Il existe donc un besoin pour une composition cosmétique de maquillage des fibres kératiniques, sans cires, qui présente une teneur en solides élevée tout en présentant, conservant, de préférence, un indice de consistance faible.

Il existe encore un besoin pour une composition cosmétique de maquillage des fibres kératiniques, par exemple une composition de maquillage des yeux, telle qu'un mascara, exempte de cires, qui présente d'excellentes propriétés d'application lors de son application, à savoir qui permette notamment une application facile et homogène et qui procure aussi, simultanément, d'excellents résultats quant au maquillage final obtenu en particulier un bon effet volumateur. Le but de l'invention est de fournir une composition cosmétique de maquillage ou de soin des fibres kératiniques qui réponde, entre autres, à ces besoins.

Le but de l'invention est encore de fournir une composition de maquillage ou de soin des fibres kératiniques, telle qu'une composition de mascara qui résolve les problèmes des compositions de l'art antérieur et qui ne présente pas les inconvénients, limitations, défauts et désavantages des compositions de l'art antérieur.

Ce but, et d'autres encore, sont atteints conformément à l'invention par une composition cosmétique non rincée de maquillage ou de soin des fibres kératiniques, comprenant au moins une phase grasse comprenant au moins un agent structurant constitué par l'association d'un composé particulier et d'au moins une huile, ladite composition ne contenant pas de cires et présentant une teneur en solides définie par un extrait sec en solides supérieur à 45 % en poids.

Les compositions selon l'invention, de manière surprenante, présentent, bien qu'elles ne contiennent pas de cire, une teneur en solide élevée, à savoir supérieure à 45 % en poids, qui n'a jamais pu être obtenue dans les compositions non-rincées de l'art antérieur sans que ne soient incorporées de cires dans celles-ci.

Un autre avantage procuré par l'absence de cires est qu'elle favorise encore l'aspect lisse, homogène et non granuleux du dépôt, mais qu'elle permet ainsi d'augmenter encore plus la teneur en solides car l'influence des cires sur la consistance n'intervient pas.

Avantageusement, la composition selon l'invention a une consistance inférieure à 1 000 Pa.

Ainsi, les compositions sans cires selon l'invention présentent avantageusement une combinaison d'une teneur en solides élevées, à savoir supérieure à 45 % en poids et d'un indice de consistance faible, à savoir inférieur à 1 000 Pa, qui n'est jamais décrite, ni suggérée dans l'art antérieur.

L'indice de consistance est une grandeur qui permet de rendre compte de la consistance globale du produit.

Du fait de la forte teneur globale en solides dans la composition finale et, avantageusement, d'un indice de consistance, faible, satisfaisant, généralement de l'ordre de celui des mascaras connus de l'art antérieur, voire inférieur à celui des mascaras connus, on obtient de manière surprenante une application facile, un dépôt homogène, allant de pair avec un effet volumateur et séparant satisfaisant.

En d'autres termes, dans le cas où la consistance est inférieure à 1 000 Pa, la composition selon l'invention permet pour la première fois, en conséquence de la combinaison de deux paramètres spécifiques, se trouvant chacun dans une plage spécifique d'allier, de combiner d'excellentes propriétés d'applications de la composition à d'excellentes propriétés du dépôt, du maquillage obtenu avec cette composition.

Les compositions selon l'invention résolvent les problèmes des compositions de l'art antérieur et ne présentent pas les défauts, limitations et désavantages des compositions de l'art antérieur.

En particulier, les compositions de l'invention triomphent du préjugé largement répandu de l'art antérieur selon lequel il n'était pas possible d'obtenir une composition non rincée, par exemple de maquillage, notamment de maquillage des yeux, qui soit à la fois exempte de cires et qui présente une forte teneur en solides.

Dans le cas où la consistance de la composition, selon l'invention, est inférieure à 1 000 Pa, les compositions, selon l'invention, triomphent, en outre, du préjugé selon lequel il n'était pas possible d'obtenir une composition de maquillage sans cires qui ait une forte teneur en solide et, simultanément, une consistance satisfaisante pour une application facile et homogène associée à un effet volumateur et séparant satisfaisant.

En outre, la composition selon l'invention est stable, même sur une longue durée et homogène. Avantageusement l'extrait sec en solides est supérieur à 46 % en poids, de préférence supérieur à 47 % en poids, de préférence encore supérieur à 48 % en poids, et mieux supérieur à 50 % en poids.Notamment, l'extrait sec de la composition est inférieur à 85 % en poids, de préférence inférieur à 75 %, et mieux inférieur à 65 %.

Selon l'invention, plus la teneur en solides, définie par l'extrait sec en solide, est élevée, plus l' effet volumateur est important, tandis que l'application demeure toujours facile et le dépôt homogène, même à des teneurs en solides si élevées.

Avantageusement, l'indice de consistance est de 1 à 900, de préférence de 10 à 800, dans ces plages préférées, les propriétés d'application sont encore améliorées et vont toujours de pair avec un effet volumateur amélioré, voire un effet séparant également amélioré.

La composition selon l'invention comprend au moins une phase grasse comprenant au moins un agent structurant particulier, spécifique qui peut, de manière surprenante, être incorporé dans la composition, même à des teneurs très élevées, pouvant aller, par exemple, jusqu'à 60 % en poids de la composition sans augmenter de façon importante l'indice de consistance ou provoquer de prise en masse de la composition.

De manière totalement surprenante, l'utilisation, en lieu et place des cires utilisées dans l'art antérieur, de l'agent structurant particulier, selon l'invention, ne produit aucune augmentation de la consistance et permet donc d'atteindre des teneurs globales en solides beaucoup plus importantes que dans l'art antérieur.

La phase grasse peut former une phase continue de la composition. En particulier, la composition selon l'invention peut être anhydre.

La phase grasse totale de la composition peut représenter de 10 à 60 %, de préférence de 15 à 50 %, de préférence encore de 20 à 40 % du poids total de la composition.

L'incorporation d'un tel agent structurant spécifique en partie ou en totalité dans la phase grasse est possible sans augmenter fortement l'indice de consistance et il est ainsi possible d'atteindre, grâce à ces agents structurants spécifiques, des teneurs en solides supérieures à 45 % - beaucoup plus importantes que dans l'art antérieur, sans mettre en oeuvre de cires.

L'incorporation dans la phase grasse de la composition de cet agent structurant spécifique rend possible, de manière surprenante, l'obtention d'une forte teneur en solides combinée avantageusement à une consistance satisfaisante et donc l'association d'une application facile et homogène et d'un effet épaississant et séparant satisfaisant.

En d'autres termes, il a été possible selon l'invention d'obtenir, sans avoir recours à des cires, des compositions stables et homogènes avec un extrait sec en solide supérieur à 45 %, en utilisant un agent structurant spécifique. Avantageusement, on a pu conserver par ailleurs, de manière surprenante, un indice de consistance inférieur à 1 000 Pa.

Ledit agent structurant est défini par des propriétés de collant et de dureté déterminées Le dit agent structurant est caractérisé par :
- une valeur de collant ≥ 0,1 N.s, notamment de 0,1 à 30 N.s **;** de préférence ≥ 0,5 N.s, notamment de 0,5 N.s à 20 N.s ; mieux ≥ 0,8 N.s, notamment de 0,8 à 10 N.s ; et encore mieux ≥ 1, notamment de 1 à 5 N.s ;
- une valeur de dureté ≤ 30 MPa, notamment entre 0,01 à 30 MPa ; de préférence entre 0,05 à 25 MPa **;** mieux entre 0,1 et 20 MPa.

Un autre éventuel avantage de l'utilisation de cet agent structurant spécifique dans les compositions de l'invention est qu'il permet également une meilleure accroche sur la matière kératinique, telle que le cil, homogène et rapide, du fait de son caractère éventuellement collant.

Il s'est avéré que l'incorporation dans la phase grasse de la composition de l'invention d'un tel agent défini par des valeurs spécifiques de certains paramètres permettrait précisément d'obtenir sans cires des taux de solides élevés, éventuellement en association avec les consistances faibles recherchées, et de ce fait la combinaison de propriétés et d'effets voulus.

L'agent structurant ou les agents structurants est (sont) choisi(s) parmi les associations d'un composé particulier avec au moins une huile.

La présence d'une huile est particulièrement appropriée pour une composition de soin ou de traitement cosmétique des fibres kératiniques, en particulier des cils.

Le composé particulier peut être choisi parmi les polymères semi-cristallins ; les agents rhéologiques de phase grasse, tels que les polymères de type polyamide, les silices hydrophobes ; et leurs mélanges.

On précise que, selon l'invention, dans le cas des associations du composé particulier avec une huile, on entend par « huile »,un corps gras liquide à température ambiante.

On précise en outre que par « composé volatil » par exemple « huile volatile », on entend, au sens de l'invention, tout composé (ou milieu non aqueux) susceptible de s'évaporer au contact de la peau ou de la fibre kératinique en moins d'une heure, à température ambiante et pression atmosphérique. Le composé volatil est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa (10⁻³ à 300 mm de Hg) , en particulier allant de 1,3 Pa à 13 000 Pa (0,01 à 100 mm de Hg), et plus particulièrement allant de 1,3 Pa à 1 300 Pa (0,01 à 10 mm de Hg).

Par opposition, on entend par « composé non volatil » par exemple « huile non volatile », un composé restant sur la peau ou la fibre kératinique à température ambiante et pression atmosphérique, au moins plusieurs heures et ayant notamment une pression de vapeur inférieure à 10⁻³ mm de Hg (0,13 Pa).

L'huile peut être choisie parmi les huiles hydrocarbonées et/ou siliconées et/ou fluorées volatiles et non volatiles et leurs mélanges. Ces huiles peuvent être d'origine animale, végétale, minérale ou synthétique. Par « huile hydrocarbonée », on entend une huile comportant principalement des atomes de carbone et d'hydrogène et éventuellement une ou plusieurs fonctions choisies parmi les fonctions hydroxyle, ester, éther, carboxylique. A titre d'exemple d'huile utilisable dans l'invention, on peut citer :
- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam ;
- les esters et les éthers de synthèse notamment d'acides gras comme les huiles de formule R₁COOR₂ dans laquelle R₁ représente le reste d'un acide gras supérieur comportant de 1 à 40 atomes de carbone et R₂ représente une chaîne hydrocarbonée contenant de 1 à 40 atomes de carbone avec R₁ + R₂ ≥ 10 comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates ;
- leurs mélanges.

De préférence, l'huile a une masse moléculaire supérieure ou égale à 250 g/mol notamment entre 250 et 10000 g/mol, de préférence supérieure ou égale à 300g/mol, notamment entre 300 et 8000 g/mol et mieux, supérieure ou égale à 400 g/mol, notamment entre 400 et 5000 g/mol.

Généralement dans la phase grasse, le rapport du ou des huile(s) au(x) composé(s) particulier(s) est de 10/90 à 90/10, de préférence de 20/80 à 80/20 et de préférence encore de 30/70 à 70/30.

Cette huile peut être choisie parmi :
- les polybutylènes tels que l'INDOPOL H-100 (de masse molaire ou MM=965 g/mol), l'INDOPOL H-300 (MM=1340 g/mol), l' INDOPOL H-1500 (MM=2160g/mol) commercialisés ou fabriqués par la société AMOCO ;
- les polyisobutylènes hydrogénés tels que le PANALANE H-300 E commercialisé ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol) ;
- Les polydécènes et les polydécènes hydrogénés tels que : le PURESYN 10 (MM=723 g/mol), le PURESYN 150 (MM=9200 g/mol) commercialisé ou fabriqués par la société MOBIL CHEMICALS,
- les esters tels que
- les esters d'acides gras linéaires ayant un nombre total de carbone allant de 30 à 70 comme le tétrapélargonate de pentaérythrityle (MM=697,05 g/mol),
- les esters hydroxylés tels que le malate de diisostéaryle (MM= 639 g/mol),
- les esters aromatiques tels que le tridecyl trimellitate (MM=757,19 g/mol),
- les esters d'alcool gras ou d'acides gras ramifiés en C24-C28 tels que ceux décrits dans la demande EP-A-0 955 039, et notamment le citrate de triisocétyle (MM= 865 g/mol), le tétraisononanoate de pentaérythrityle (MM=697,05g/mol), le triisostéarate de glycéryle (MM=891,51 g/mol), le tri décyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538,66 g/mol),
- les huiles d'origine végétale telles que l'huile de sésame (820,6 g/mol),
- et leurs mélanges.

On précise que, selon l'invention, dans le cas des associations susmentionnées, on entend par "polymère semi-cristallin", des polymères comportant une partie cristallisable, chaîne pendante cristallisable ou séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin selon l'invention a une température de fusion supérieure ou égale à 30 °C (notamment allant de 30 °C à 80 °C), de préférence allant de 30 °C à 60 °C. Cette température de fusion est une température de changement d'état du premier ordre.

Cette température de fusion peut être mesurée par toute méthode connue et en particulier à l'aide d'un calorimètre à balayage différentiel (D.S.C) .

De façon avantageuse, le ou les polymères semi-cristallins auxquelles s'applique l'invention présentent une masse moléculaire moyenne en nombre supérieure ou égale à 1 000.

De façon avantageuse, le ou les polymères semi-cristallins de la composition de l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être chaîne comportant au moins 6 atomes de carbone.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. En outre, ils ne comportent pas de squelette polysaccharidique. De façon générale, les motifs (chaînes ou séquences) cristallisables des polymères semi-cristallins selon l'invention, proviennent de monomère (s) à séquence (s) ou chaîne (s) cristallisable(s), utilisé(s) pour la fabrication des polymères semi-cristallins.

Selon l'invention, le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

Les polymères semi-cristallins utilisables dans l'invention sont en particulier :
- les copolymères séquences de polyoléfines à cristallisation contrôlée, notamment ceux dont les monomères sont décrits dans EP-A-0 951 897.
- les polycondensats et notamment de type polyester, aliphatique ou aromatique ou copolyester aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré(s) tels que décrits dans le document WO-A-01/19333,
- et leurs mélanges.

Dans ces deux derniers cas, la ou les chaînes latérales ou séquences cristallisables sont hydrophobes.

### A) Polymères semi-cristallins à chaînes latérales cristallisables

On peut citer en particulier ceux définis dans le document US-A-5156911 et WO-A-01/19333. Ce sont des homopolymères ou copolymères comportant de 50 à 100 % en poids de motifs résultant de la polymérisation d'un ou plusieurs monomères porteurs de chaîne latérale hydrophobe cristallisable.
. Ces homo- ou co-polymères sont de toute nature du moment qu'ils présentent les conditions indiquées précédemment.

Ils peuvent résulter :
- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

D'une façon générale, ces polymères sont choisis notamment parmi les homopolymères et copolymères résultant de la polymérisation d'au moins un monomère à chaîne(s) cristallisable(s) qui peut être représenté par la formule X : 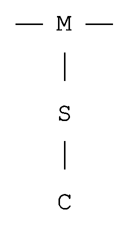 avec M représentant un atome du squelette polymérique, S représentant un espaceur, C représentant un groupe cristallisable.

Les chaînes « -S-C » cristallisables peuvent être aliphatiques ou aromatiques, éventuellement fluorées ou perfluorées. « S » représente notamment un groupe (CH₂) ₙ ou (CH₂CH₂O)ₙ ou (CH₂O), linéaire ou ramifié ou cyclique, avec n entier allant de 0 à 22. De préférence « S » est un groupe linéaire. De préférence, « S » et « C » sont différents.

Lorsque les chaînes « -S-C » cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyle en C₁₄-C₂₄. Lorsqu'il s'agit de chaînes alkyle fluorées ou perfluorées, elles comportent au moins 6 atomes de carbone fluorés et notamment au moins 11 atomes de carbone dont au moins 6 atomes de carbone sont fluorés.

Comme exemple de polymères ou copolymères semi-cristallins à chaîne (s) cristallisable (s) , on peut citer ceux résultant de la polymérisation d'un ou plusieurs monomères suivants : les (méth)acrylates d' alkyle saturés avec le groupe alkyle en C₁₄-C₂₄, les (méth)acrylates de perfluoroalkyle avec un groupe alkyle perfluoro en C₁₁-C₁₅, les N-alkyl (méth)acrylamides avec le groupe alkyle en C₁₄ à C₂₄ avec ou sans atome de fluor, les esters vinyliques à chaînes alkyle ou perfluoro (alkyle) avec le groupe alkyle en C₁₄ à C₂₄ (avec au moins 6 atomes de fluor pour une chaîne perfluoro alkyle), les éthers vinyliques à chaînes alkyle ou perfluoro (alkyle) avec le groupe alkyle en C₁₄ à C₂₄ et au moins 6 atomes de fluor pour une chaîne perfluoro alkyle, les alphaoléfines en C₁₄ à C₂₄ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50% de groupes Y ou Z résultant de la copolymérisation :
α ) de Y qui est un monomère polaire ou non polaire ou un mélange des deux :
   . Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un N,N-dialkyl(méth)acrylamide comme par exemple le N,N-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
   . Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou styrène substitué par un groupe alkyle en C₁ à C₁₀, comme l'α-méthylstyrène, un macromonomère du type polyorganosiloxane à insaturation vinylique.

Par "alkyle», on entend au sens au sens de l'invention un groupement saturé notamment en C₈ à C₂₄, sauf mention exprès, et mieux en C₁₄ à C₂₄.

β) de Z qui est un monomère polaire ou un mélange de monomères polaires. Dans ce cas, Z a la même définition que le "Y polaire" défini ci-dessus.

De préférence, les polymères semi-cristallins à chaîne latérale cristallisable sont des homopolymères d' alkyl (méth) acrylate ou d'alkyl(méth)acrylamide avec un groupe alkyle tel que défini ci-dessus, et notamment en C₁₄-C₂₄, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

### B) Les polymères portant dans le squelette au moins une séquence cristallisable

Ces polymères sont notamment des copolymères séquencés constitués d'au moins 2 séquences de nature chimique différente dont l'une est cristallisable.
- On peut utiliser les polymères séquencés définis dans le brevet US-A-5 156 911 ;
- Les copolymères séquencés d'oléfine ou de cyclooléfine à chaîne cristallisable comme ceux issus de la polymérisation séquencée de :
   . cyclobutène, cyclohexène, cyclooctène, norbornène (c'est-à-dire bicyclo (2,2,1) heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
   . avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
   . et en particulier les copoly(éthylène/norbornène) blocs et les terpolymères (éthylène/propylène/éthylidène-norbornène) blocs. On peut aussi utiliser ceux résultants de la copolymérisation séquencée d'au moins 2 α-oléfines en C₂-C₁₆ et mieux en C₂-C₁₂ et encore mieux en C₄-C₁₂ tels que ceux cités précédemment et en particulier les bipolymères séquencés d'éthylène et d'1-octène
- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
   . Séquence cristallisable par nature : a) polyester comme les poly(alkylène téréphtalate), b) polyoléfine comme les polyéthylènes ou polypropylènes.
   . Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe distinctes, on peut citer :
α) les copolymères séquences poly(ε-caprolactone)-b-poly(butadiène), utilisés de préférence hydrogénés, tels que ceux décrits dans l'article "Melting behavior of poly(ε-caprolactone)-block-polybutadiène copolymers" de S. Nojima, Macromolécules, 32, 3727-3734 (1999).
β) les copolymères séquences poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995) .
γ) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et " Polymer agregates with crystalline cores : the system poly(ethylene)-poly(ethylene-propylene)" de P. Richter et al., Macromolécules, 30, 1053-1068 (1997).
δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins de la composition de l'invention peuvent être ou non réticulés en partie du moment que le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.

De préférence, les polymères semi-cristallins de la composition selon l'invention sont non réticulés.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en C₁₄ à C₂₄, les (méth)acrylates de perfluoroalkyle en C₁₁ à C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alphaoléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ éventuellement fluoré, qui peut être représenté par la formule suivante : 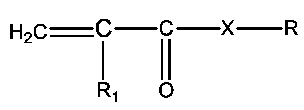 dans laquelle R₁ est H ou CH₃, R représente un groupe alkyle en C₁-C₁₀ éventuellement fluoré et X représente O, NH ou NR₂, où R₂ représente un groupe alkyle en C₁-C₁₀ éventuellement fluoré.

Selon un mode plus particulier de réalisation de l'invention, le polymère est issu d'un monomère à chaîne cristallisable choisi parmi les (méth)acrylates d'alkyle saturés en C₁₄ à C₂₂.

A titre d'exemple particulier de polymère semi-cristallin structurant utilisable dans la composition selon l'invention, on peut citer les produits Intelimer® de la société Landec décrits dans la brochure " Intelimer® polymers", Landec IP22 (Rev. 4-97). Ces polymères sont sous forme solide à température ambiante (25°C). Ils sont porteurs de chaînes latérales cristallisables et présentent la formule X précédente.

Les polymères semi-cristallins peuvent être notamment :
ceux décrits dans les exemples 3, 4, 5, 7, 9, 13 du brevet US-A-5 156 911 à groupement -COOH, résultant de la copolymérisation d'acide acrylique et d' alkyl (méth) acrylate en C₅ à C₁₆ et plus particulièrement de la copolymérisation :
   . d'acide acrylique, d'hexadécylacrylate et d'isodécylacrylate dans un rapport pondéral 1/16/3,
   . d'acide acrylique et de pentadécylacrylate dans un rapport pondéral 1/19,
   . d'acide acrylique, d'hexadécylacrylate, éthylacrylate dans un rapport pondéral 2,5/76,5/20,
   . d'acide acrylique, d'hexadécylacrylate et de méthylacrylate dans un rapport pondéral 5/85/10,
   . d'acide acrylique et de octadécylméthacrylate dans un rapport pondéral 2,5/97,5,
   . d'hexadécylacrylate, de monométhyl éther de méthacrylate polyéthylèneglycol à 8 motifs d'éthylèneglycol, et d'acide acrylique dans un rapport pondéral 8,5/1/0,5.

On peut aussi utiliser le polymère de structure « O » de National Starch tel que celui décrit dans le document US-A-5 736 125 de point de fusion 44°C ainsi que les polymères semi-cristallins à chaînes pendantes cristalisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP tels que décrits dans le document US-A-5 519 063 ou EP-A-550745, de température de fusion respectivement de 40°C et 38°C.

On peut aussi utiliser les polymères semi-cristallins obtenus par copolymérisation de l'acrylate de béhényle et de l'acide acrylique ou de NVP tels que décrits dans les documents US-A-5519063 et EP-A-550745, de température de fusion respectivement de 60°C et 58°C.

De préférence, les polymères semi-cristallins ne comportent pas de groupement carboxylique.

Les autres associations susmentionnées peuvent comprendre l'association d'un agent rhéologique avec une huile.

Cet agent rhéologique est capable d'épaissir et/ou gélifier la phase huile. Il peut être présent en une quantité efficace pour augmenter la viscosité de cette phase, notamment jusqu'à l'obtention d'un gel solide, à savoir un produit ne s'écoulant pas sous son propre poids.

Cet agent rhéologique est avantageusement choisi parmi les gélifiants lipophiles, les organogélateurs et leurs mélanges.

Le gélifiant lipophile peut être organique ou minéral, polymérique ou moléculaire.

Comme gélifiant lipophile minéral, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium .

On peut églement citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812® " par la société Degussa, "CAB-O-SIL TS-530®^{"} par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6^{ème} édition, 1995). Elles sont par exemple commercialisées sous les références "AEROSIL R972® ", "AEROSIL R974® " par la société Degussa, "CAB-O-SIL TS-610® ", " CAB-O-SIL TS-720® " par la société Cabot.

La silice pyrogénée hydrophobe présente de préférence une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ; l'éthylcellulose comme celles vendues sous le nom d'Ethocel par Dow Chemical ; les polyamides tels que les copolymères d'un diacide en C₃₆ condensé sur l'éthylène diamine de masse moléculaire moyenne en poids d'environ 6000 tels que les composés commercialisés par la société Arizona Chemical sous les noms Uniclear 80 et Uniclear 100, les gommes notamment siliconées comme les PDMS ayant une viscosité > 100 000 centistokes, les galactommananes comportant de un à six et mieux de deux à quatre groupes hydroxyle par ose, substitués par une chaîne alkyle saturée ou non, comme la gomme de guar alkylée par des chaînes alkyle en C₁ à C₆ et mieux en C₁ à C₃ et leurs mélanges.

Comme gélifiant lipophile préféré, on utilise des gélifiants organique moléculaires non polymériques, également appelés organogélateurs, qui sont des composés dont les molécules sont capables d'établir entre elles des interactions physiques conduisant à une auto-agrégation des molécules avec formation d'un réseau supra-moléculaire 3D qui est responsable de la gélification de la phase grasse liquide.

Par "phase grasse liquide", on entend, au sens de l'invention, une phase grasse liquide à température ambiante (25° C) et pression atmosphérique (760 mm de Hg, soit 105 Pa), composée d'un ou plusieurs corps gras liquides température ambiante, appelés aussi huiles, généralement compatibles entre eux.

Le réseau supra-moléculaire peut résulter de la formation d'un réseau de fibrilles (dues aux empilements ou agrégations de molécules d'organogélateur), immobilisant les molécules de la phase grasse liquide.

L'aptitude à former ce réseau de fibrilles, et donc à gélifier, dépend de la nature (ou classe chimique) de l'organogélateur, de la nature des substituants portés par ses molécules pour une classe chimique donnée et de la nature de la phase grasse liquide.

Les interactions physiques sont diverses mais excluent la co-cristallisation. Ces interactions physiques sont en particulier des interactions du type interactions hydrogènes auto-complémentaires, interactions π entre cycles insaturés, interactions dipolaires, liaisons de coordination avec des dérivés organométalliques et leurs associations. En général, chaque molécule d'un organogélateur peut établir plusieurs types d'interactions physiques avec une molécule voisine. Aussi, avantageusement, les molécules des organogélateurs selon l'invention comportent au moins un groupement capable d'établir des liaisons hydrogènes et mieux au moins deux groupements capables d'établir des liaisons hydrogène, au moins un cycle aromatique et mieux aux moins deux cycles aromatiques, au moins une ou plusieurs liaisons à insaturation éthylénique et/ou au moins un ou plusieurs carbones asymétriques. De préférence, les groupements capables de faire des liaisons hydrogènes sont choisis parmi les groupements hydroxyle, carbonyle, amine, acide carboxylique, amide, urée, benzyle et leurs associations.

Le ou les organogélateurs selon l'invention sont solubles dans la phase grasse liquide après chauffage jusqu'à obtention d'une phase liquide homogène transparente. Ils peuvent être solides ou liquides à température ambiante et pression atmosphérique.

Le ou les organogélateurs moléculaires utilisables dans la composition selon l'invention sont notamment ceux décrits dans le document "Specialist Surfactants", édité par D. Robb de 1997, p.209-263, chapitre 8 de P. Terech, les demandes européennes EP-A-1068854 et EP-A-1086945 ou encore dans la demande WO-A-02/47031.

On peut notamment citer parmi ces organogélateurs, les amides d'acides carboxyliques en particulier les acides tri-carboxyliques comme les cyclohexanetricarboxamides (voir la demande de brevet européen EP-A-1068854), les diamides ayant des chaînes hydrocarbonées contenant chacune de 1 à 22 atomes de carbone, par exemple de 6 à 18 atomes de carbone, lesdites chaînes étant non substituées ou substituées avec au moins un substituant choisi parmi les groupes ester, urée et fluoro (voir la demande EP-A-1086945) et notamment les diamides résultant de la réaction du diaminocyclohexane, en particulier du diaminocyclohexane sous forme trans, et d'un chlorure d'acide comme par exemple le N,N'-bis (dodécanoyl)-1,2-diaminocyclohexane, les amides de N-acylamino acides comme les diamides résultant de l'action d'un N-acylamino acide avec des amines comportant de 1 à 22 atomes de carbone, comme par exemple ceux décrits dans le document WO-93/23008 et notamment les amides de l'acide N-acylglutamique où le groupe acyle représente une chaîne alkyle en C₈ à C₂₂ tels que le dibutylamide de l'acide N-Lauroyl-L-glutamique, fabriqué ou commercialisé par la société Ajinomoto sous la dénomination GP-1 et leurs mélanges.

Les compositions peuvent contenir de 10 à 60% de l'agent structurant. De préférence, la composition contient de 15% à 50% en poids, mieux de 20% à 40% d'agent structurant, ce qui permet d'atteindre des teneurs globales en solides dans la composition > 45%, de préférence >46%, mieux > 47%, encore mieux > 48% voire > 50% tout en conservant un indice de consistance satisfaisant < 1000Pa, de préférence entre 1 et 900 Pa et mieux entre 10 et 800Pa.

Les compositions classiques de maquillage des yeux, tels que les mascaras présentent généralement un indice de consistance de cet ordre de grandeur mais pour des extraits secs, qui sont inférieurs à 45% et souvent inférieurs à 40% ; par exemple, entre 30% et 40% ; ce qui limite l'effet volumateur.

Un exemple (A) d'un agent structurant selon l'invention constitué d'un polymère semi-cristallin associé à une huile est le suivant :
Phase grasse = mélange polybutène /cop.acrylate de stéaryle N-vinyl pyrrolidonne (40/60)de point de fusion de 56°C.
Phase grasse = mélange polybutène(1) / cop.acrylate de stéaryle N-vinyl pyrrolidonne(2) (40/60)de point de fusion de 56°C.
   (1) : Indopol H 100 de la société AMOCO
   (2) : Polymère basique de point de fusion de 56°C préparé selon le mode opératoire suivant.

Dans un réacteur d'1l muni d'une agitation centrale avec ancre, d'un réfrigérant et d'un thermomètre, on introduit 120 g de cyclohexane que l'on chauffe de la température ambiante à 80°C en 45 min. A 80°C, on introduit en 2h le mélange C₁ suivant :
40 g de cyclohexane + 4 g de Triganox 141 [2,5 bis (2-éthyl hexanoyl peroxy) - 2,5 - diméthyl hexane].

30 min après le début de la coulée du mélange C₁, on introduit en 1h30 le mélange C₂ constitué de :
190 g d'acrylate de stéaryle + 10 g de N-vinyl pyrrolidone + 400 g de cyclohexane.

A la fin des deux coulées, on laisse agir 3h supplémentaires à 80°C puis on distille à pression atmosphérique la totalité du cyclohexane présent dans le milieu réactionnel.

On obtient alors le polymère à 100 % en poids en matière active .

Sa masse moléculaire moyenne en poids M_{w} est de 38 000 exprimée en équivalent polystyrène et sa température de fusion pF est de 56°C , mesurée par D.S.C.
Collant = 2,63 N.s
Dureté = 5,84 MPa

### Protocole de mesure du collant

Dans ce qui suit, le protocole de mesure du collant est décrit pour une cire mais ce protocole s'applique aussi aux autres agents structurants notamment aux agents structurants constitués par l'association d'un composé particulier et d'une huile.

Le collant de la cire est mesuré à 20 °C à l'aide du texturomètre vendu sous la dénomination TA-TX2i par la société RHEO, équipé d'un mobile en polymère acrylique en forme de cône formant un angle de 45°, en mesurant l'évolution de la force (force de compression ou force d'étirement) (F) en fonction du temps, pendant l'opération suivante :

Le mobile est déplacé à la vitesse de 0,5 mm /s puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s. Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

Pour effectuer la mesure du collant de la cire, la cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

### Protocole de mesure de la dureté

Dans ce qui suit, le protocole de mesure de dureté est décrit pour une cire mais ce protocole s'applique aussi aux autres agents structurants notamment aux agents structurants constitués par l'association d'un composé particulier et d'une huile.

La dureté de la cire est mesuré à 20 °C à l'aide du texturomètre vendu sous la dénomination TA-TX2i par la société RHEO, équipé d'un mobile en inox en forme de cylindre d'un diamètre de 2mm., en mesurant l'évolution de la force (force de compression ou force d'étirement) (F) en fonction du temps, pendant l'opération suivante :

Le mobile est déplacé à la vitesse de 0,1 mm /s puis pénètre dans la cire jusqu'à une profondeur de pénétration de 0.3 mm. Lorsque le mobile a pénétré dans la cire à la profondeur de 0.3 mm, le mobile est maintenu fixe pendant 1 seconde (correspondant au temps de relaxation) puis est retiré à la vitesse de 0,1 mm/s. Pendant le temps de relaxation, la force (force de compression) décroît fortement jusqu'à devenir nulle puis, lors du retrait du mobile, la force (force d'étirement) devient négative pour ensuite croître à nouveau vers la valeur 0. La dureté correspond à la force de compression maximale mesurée entre la surface du mobile et la cire au moment de leur mise en contact. La valeur de cette force est exprimée en MPa.

Pour effectuer la mesure de la dureté de la cire, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté.

### Protocole de mesure de l'indice de consistance

La caractérisation des compositions selon l'invention se fait à l'aide de l'analyseur de texture sous la dénomination TA-TX2i par la Société RHEO.

L'essai consiste à mettre en contact une sonde cylindrique en inox de 12 mm de diamètre avec le produit (récipient cylindrique de diamètre = 35 mm et profondeur = 15 mm rempli de produit dont la surface est arasée de façon à obtenir une surface bien plane).

La mesure est répétée trois fois par produit.

La mesure de l'indice de consistance se fait lors de la phase de contact de la sonde avec le produit. Cette phase se fait à un déplacement constant de 0,2 mm. La sonde s'enfonce donc dans le produit jusqu'à cette profondeur de 0,2 mm à une vitesse de 10 mm/s. La force (ou contrainte) mesurée à ce moment donne l'indice de consistance (en Pa) du produit. La sonde est alors maintenue dans cette position pendant une seconde.

### Protocole de mesure de la teneur en solides

Consiste en une mesure de l'extrait sec du jus de mascara réalisé sur une balance Mettler Toledo HG 53 (Halogen Moisture Analyzer).

Un échantillon de mascara (2-3g) est déposé sur une coupelle en aluminium et subit une température de 120°C pendant 60 minutes. La mesure de l'extrait sec correspond au suivi de la masse de l'échantillon en fonction du temps .La teneur finale en solides est donc le pourcentage de la masse finale (au bout de 60 min) par rapport à la masse initiale : ES = (masse finale /masse initiale) X 100.

La composition, selon l'invention, est une composition de maquillage, une base de maquillage dite encore « base coat », une composition dite « top-coat » à appliquer sur un maquillage, ou une composition de traitement cosmétique, de soin, des fibres kératiniques.

La composition selon l'invention s'applique plus particulièrement aux cils. De ce fait, la composition de l'invention peut être une composition de revêtements des cils, notamment une composition de maquillage des cils, encore appelé mascara, une composition à appliquer sur un maquillage des cils, dite encore top-coat, ou bien encore une composition de traitement des cils, notamment des cils d'êtres humains ou des faux cils. Plus spécialement, la composition est un mascara.

La composition selon l'invention forme un milieu physiologiquement acceptable.

Dans la présente demande, on entend par "milieu physiologiquement acceptable", un milieu non toxique compatible avec les fibres kératiniques d'êtres humains, notamment les cils ou les sourcils, comme un milieu cosmétique, le milieu cosmétique pouvant être un milieu cosmétique hydrophile ou lipophile.

La composition peut comprendre de l'eau et éventuellement un ou plusieurs solvant(s) organique(s) hydrophile ( s ) , c' est-à-dire un ou des solvant(s) organique(s) miscibles à l'eau, comme les alcools et notamment des monoalcools ayant de 2 à 5 atomes de carbone comme l'éthanol, l'isopropanol ou le n-propanol, les polyols ayant de 2 à 8 atomes de carbone comme la glycérine, la diglycérine, le propylène glycol, l'éthylène glycol, le 1,3-butylène glycol, le sorbitol, le penthylène glycol, les cétones en C₃-C₄, les aldéhydes en C₂-C₄.

L'eau ou le mélange d'eau et de solvant(s) organique(s) hydrophile(s) peut être présent dans la composition selon l'invention en une teneur allant de 0,1 % à 90 % en poids, par rapport au poids total de la composition, et de préférence de 0,1 % à 60 % en poids.

Selon un autre mode de réalisation, la phase grasse telle que définie précédemment peut former une phase continue de la composition. En particulier, la composition selon l'invention peut être anhydre.

La composition selon l'invention peut comprendre au moins un composé gras pâteux à température ambiante. Par "corps gras pâteux" au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le corps gras pâteux peut être présent dans la composition selon l'invention en une teneur allant de 0,01 à 60% en poids, par rapport au poids total de la composition, de préférence allant de 0,5 à 45 % en poids, et mieux allant de 2 % à 30 % en poids, dans la composition.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges.
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition selon l'invention peut comprendre au moins un polymère filmogène.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques comme les cils.

Le ou les polymères filmogènes qui peuvent être présents dans la composition de l'invention sont différents du « polymère semi-cristallin » tel qu'il a été défini plus haut.

De préférence, le polymère filmogène ne comprend pas de motifs cristallisables. Dans le cas où il comprendrait des motifs cristallisables, ceux-ci représenteraient moins de 30 % en poids du poids total du polymère.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α, β -éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth) acrylique (encore appelé les (méth) acrylates) , notamment des (méth)acrylates d'alkyle, en particulier d'alkyle en C₁-C₃₀, de préférence en C₁-C₂₀, des (méth)acrylates d'aryle, en particulier d'aryle en C₆-C₁₀, des (méth)acrylates d'hydroxyalkyle, en particulier d'hydroxyalkyle en C₂-C₆ .

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N -alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺ , Fe ³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane diméthanol, acide isophtalique, acide sulfoisophtalique.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être un polymère hydrosoluble et peut être présent dans une phase aqueuse de la composition ; le polymère est dons solubilisé dans la phase aqueuse de la composition. Comme exemples de polymères filmogènes hydrosolubles, on peut citer :
- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
   . les gommes arabiques, la gomme de guar, les dérivés du xanthane, la gomme de karaya ;
   . les alginates et les carraghénanes ;
   . les glycoaminoglycanes, l'acide hyaluronique et ses dérivés ;
   . la résine shellac, la gomme de sandaraque, les dammars, les élémis, les copals ;
   . l'acide désoxyribonucléique ;
   . les muccopolysaccharides tels les chondroïtines sulfate,
et leurs mélanges.

Selon une autre variante de réalisation de la composition selon l'invention, le polymère filmogène peut être un polymère solubilisé dans une phase grasse liquide comprenant des huiles ou solvants organiques tels que ceux décrits précédemment (on dit alors que le polymère filmogène est un polymère liposoluble). Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides à température ambiante, appelés aussi huiles, généralement compatibles entre eux.

De préférence, la phase grasse liquide comprend une huile volatile, éventuellement en mélange avec une huile non volatile, les huiles pouvant être choisies parmi les huiles citées précédemment.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui ont pour but qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de la copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate d'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂-C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Selon encore une autre variante de réalisation de la composition de l'invention, le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations « Neocryl XK-90® », « Neocryl A-1070® », « Neocryl A-1090® », « Neocryl BT-62® », « Neocryl A-1079® » et « Neocryl A-523® » par la société AVECIA-NEORESINS, « Dow Latex 432® » par la société DOW CHEMICAL, « Daitosol 5000 AD® » par la société DAITO KASEY KOGYO; ou encore les dispersions aqueuses de polyuréthane vendues sous les dénominations « Neorez R-981® » et « Neorez R-974® » par la société AVECIA-NEORESINS, les « Avalure UR-405® », « Avalure UR-410® », « Avalure UR-425® », « Avalure UR-450® », « Sancure 875® », « Sancure 861® », « Sancure 878® » et « Sancure 2060® » par la société GOODRICH, « Impranil 85® » par la société BAYER, « Aquamere H-1511® » par la société HYDROMER ; les sulfopolyesters vendus sous le nom de marque « Eastman AQ® » par la société EASTMAN CHEMICAL PRODUCTS, les dispersions vinyliques comme le « Mexomère PAM » et aussi les dispersions acryliques dans l'isododécane comme le « Mexomère PAP » par la société CHIMEX.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le **β**-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition selon l'invention peut en outre comprendre des fibres qui permettent une amélioration de l'effet allongeant, en particulier dans le cas où la composition selon l'invention est un mascara.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

Les fibres peuvent être celles utilisées dans la fabrication des textiles et notamment des fibres de soie, de coton, de laine, de lin, des fibres de cellulose - notamment extraites notamment du bois, des légumes ou des algues -, de rayonne, de polyamide (Nylon® ), de viscose, d'acétate notamment d'acétate de rayonne, de poly-(p-phénylène-téréphtalamide) (ou d'aramide) notamment de Kevlar® , de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

On peut aussi utiliser les fibres utilisées en chirurgie comme les fibres synthétiques résorbables préparées à partir d'acide glycolique et caprolactone ("Monocryl" de chez Johnson & Johnson) ; les fibres synthétiques résorbables du type copolymère d'acide lactique et d'acide glycolique ("Vicryl" de chez Johnson & Johnson) ; les fibres de polyester téréphtalique ("Ethibond" de chez Johnson & Johnson) et les fils d'acier inoxydable ("Acier" de chez Johnson & Johnson) notamment pour une application en vernis à ongles.

Par ailleurs, les fibres peuvent être traités ou non en surface, enrobées ou non.

Les fibres utilisables dans la composition selon l'invention sont préférentiellement des fibres de polyamide, de cellulose, de poly-p-phénylène téréphtalamide ou de de polyéthylène. Leur longueur (L) peut aller de 0,1 mm à 5 mm, de préférence de 0,25 mm à 1, 6 mm et leur diamètre moyen peut aller de 1 µm à 50 µm. En particulier, on peut utiliser les fibres de polyamide commercialisées par les Etablissements P. Bonte sous le nom de "Polyamide 0.9 Dtex 3 mm", ayant un diamètre moyen de 6 µm, un titre d'environ 0 . 9 dtex et une longueur allant de 0,3 mm à 5 mm. On peut aussi utiliser les fibres de celluloses (ou de rayonne) ayant un diamètre moyen de 50 µm et une longueur allant de 0,5 mm à 6 mm comme celles vendues sous le nom de "Natural rayon flock fiber RC1BE - N003 - M04" par la société Claremont Flock. On peut également utiliser des fibres de polyéthylène comme celles vendues sous le nom de "Shurt Stuff 13 099 F" par la société Mini Fibers.

La composition selon l'invention peut également comprendre des fibres dites "rigides", par opposition aux fibres citées précédemment, qui ne sont pas des fibres rigides.

Les fibres rigides peuvent être choisies parmi les fibres d'un polymère synthétique choisi parmi les polyesters, les polyuréthanes, les polymères acryliques, les polyoléfines, les polyamides, en particulier les polyamides non aromatiques et les polyimides-amides aromatiques.

Comme exemples de fibres rigides, on peut citer les fibres :
- de polyesters, telles que celles obtenues par découpe de fils vendus sous les dénominations FIBRE 255-100-R11-242T TAILLE 3 MM (section octalobée), FIBRE 265-34-R11-56T TAILLE 3 MM (section ronde), FIBRE COOLMAX 50-34-591 TAILLE 3 MM (section tétralobée) par la société DUPONT DE NEMOURS ;
- de polyamide, telles que celles vendues sous les dénominations TRILOBAL NYLON 0.120-1.8 DPF **;** TRILOBAL NYLON 0.120-18 DPF ; NYLON 0.120-6 DPF par la société Cellusuede products ; ou obtenues par découpe de fils vendus sous la dénomination FIBRE NOMEX BRAND 430 TAILLE 3 MM par la société DUPONT DE NEMOURS ;
- de polyimide-amide telles que celles vendues sous les dénomination "KERMEL", " KERMEL TECH" par la société RHODIA ;
- de poly-(p-phénylène-téréphtalamide) (ou d'aramide) notamment vendues sous la dénomination Kevlar ® par la société DUPONT DE NEMOURS ;
- des fibres à structure multicouche comprenant des couches alternées de polymères choisis parmi les polyesters, les polymères acryliques, les polyamides, tellles que celles décrites dans les documents EP-A-6921217, EP-A-686858 et US-A-5472798. De telles fibres sont vendues sous les dénominations "Morphotex", « Teijin Tetron Morphotex » par la société TEIJIN.

Les fibres rigides particulièrement préfères sont les fibres de polymide-amide aromatiques.

Des fils ou fibres de polyimide-amide, qui peuvent être utilisés pour les compositions de l'invention, sont décrits, par exemple, dans le document de R. PIGEON et P. ALLARD, Chimie Macromoléculaire Appliquée, 40/41 (1974), pages 139-158 (n° 600), ou bien encore dans les documents US-A-3 802 841, FR-A-2 079 785, EP-A1-0 360 728, EP-A-0 549 494, auquels on pourra se référer.

Les fibres de polyimide-amide aromatique préférées sont des fibres de polyimide-amide comprenant des motifs répétitif de formule : 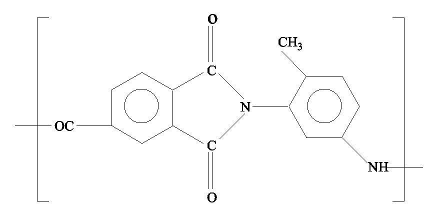 obtenu par polycondensation du toluylène diisocyanate et de l'anhydride triméllitique.

Les fibres peuvent êtres présentes dans la composition selon l'invention en une teneur allant de 0,01 % à 10 % en poids, par rapport au poids total de la composition, de préférence de 0,1 % à 5 % en poids, et mieux de 0,3 % à 3 % en poids.

La composition de l'invention peut comprendre, en outre, tout additif usuellement utilisé en cosmétique tels que les antioxydants, les charges, les conservateurs, les parfums, les neutralisants, les épaississants, les vitamines, et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique.

L'invention a trait également a un procédé cosmétique de traitement ou de maquillage des fibres kératiniques, comprenant l'application sur les dites fibres kératiniques de la composition, telle qu'elle est décrite plus haut.

L'invention concerne aussi un procédé de revêtement des cils comprenant l'application sur les cils de la composition décrite ci-dessus.

L'invention est également relative à l'utilisation de la composition, telle que décrite plus haut, pour le maquillage des fibres kératiniques ainsi qu'à l'utilisation de cette composition pour obtenir une application facile et homogène et un maquillage présentent un excellent effet volumateur et séparant.

L'invention va maintenant être décrite en référence aux exemples suivants, donnés à titre illustratif et non limitatif.

### EXEMPLES

Plusieurs compositions de mascaras ont été réalisées et caractérisées selon l'invention.

Les formulations selon l'invention sont des formulations du type émulsion phase grasse/eau définies généralement de la manière suivante :

| | |
|---|---|
| Phase grasse | x% |
| Acide stéarique | 5,82% |
| Neutralisants | 2,9 % |
| Oxyde de fer noir | 8% |
| Hydroxyethylcellulose | 0, 91% |
| Gomme arabique | 3,45% |
| Additifs, conservateurs, eau | qsp |

Dans le tableau qui suit est décrit dans l'exemple 1 une composition selon l'invention, dont on a mesuré la teneur en solides et l'indice de consistance.

Dans ce tableau, sont également indiquées, à titre de comparaison, les teneurs en solides et les indices de consistance de 2 compositions de l'art antérieur.

**Tableau**

| Essai | Teneur en solides (%) | Consistance (Pa) | Type Phase Grasse | % phase grasse = x |
|---|---|---|---|---|
| N°1 Selon invention | 56,8 | 490 | Mélange polybutène/cop. acrylate de stéaryle N-vinyl pyrrolidone (40/60) (décrit ci-dessus - EXEMPLE A) | 35 |
| Comparatif Weightless Volume wax free mascara | 28,6 | / | Sans cires | 0 |
| Comparatif Volum Express | 39 | 2030 | / | |
| Comparatif Intencils | 41,3 | 570 | / | / |

Le mascara Volum Express est vendu commercialement sous la marque Maybelline en tant que mascara volumateur.

Le mascara Intencils est vendu commercialement sous la marque Lancôme en tant que mascara volumateur.

Le mascara Weightless Volume wax free mascra est vendu commercialement sous la marque Neutrogena en tant que mascara volumateur sans cires.

Le tableau ci-dessus montre que seules les compositions de l'invention présentent, bien qu'elles ne contiennent pas de cires, une teneur en solide élevée, voire très élevée, allant de pair avec une consistance faible.

Elles permettent d'obtenir un maquillage homogène du cil, rapide et volumateur.

## Revendications

1. Composition cosmétique non rincée de maquillage ou de soin des fibres kératiniques, comprenant au moins une phase grasse comprenant au moins un agent structurant constitué par l'association d'un composé particulier et d'au moins une huile, ladite composition ne contenant pas de cires et présentant une teneur en solides définie par un extrait sec en solides supérieur à 45 % en poids.

2. Composition selon la revendication 1, qui présente un indice de consistance inférieur à 1 000 Pa.

3. Composition selon la revendication 1, présentant un extrait sec en solides supérieur à 46 % en poids, de préférence supérieure à 47 % en poids, de préférence encore supérieur à 48 % en poids, mieux supérieur à 50 % en poids.

4. Composition selon l'une quelconque des revendications précédentes présentant un indice de consistance de 1 à 900, de préférence de 10 à 800.

5. Composition selon la revendication 1, dans laquelle la phase grasse représente de 10 à 60 %, de préférence de 15 à 50 %, de préférence encore de 20 à 40 % du poids total de la composition.

6. Composition selon la revendication 1, dans laquelle l'agent structurant a une valeur de collant ≥ 0,1 N.s, notamment de 0,1 à 30 N.s ; de préférence ≥ 0,5 N.s, notamment de 0,5 N.s à 20 N.s ; mieux ≥ 0,8 N.s, notamment de 0,8 à 10 N.s ; et encore mieux ≥ 1, notamment de 1 à 5.

7. Composition selon la revendication 1, dans laquelle l'agent structurant a une valeur de dureté ≤ 30 MPa, notamment entre 0,01 à 30 MPa ; de préférence entre 0,05 à 25 MPa ; mieux entre 0,1 et 20 MPa.

8. Composition selon la revendication 1, dans laquelle le composé particulier est choisi parmi les polymères semi-cristallins ; les agents rhéologiques de la phase grasse, tels que les polymères de type polyamide, les silices hydrophobes **;** et leurs mélanges.

9. Composition selon la revendication 8, dans laquelle les polymères semi-cristallins sont choisis parmi les polyacrylates modifiés par une chaîne alkyle, solides à température ambiante et présentant un point de fusion supérieur à 30°C et inférieur à 150°C.

10. Composition selon la revendication 1, dans laquelle l'huile est choisie parmi les huiles hydrocarbonées, siliconées, et/ou fluorées, volatiles et non-volatiles, et leurs mélanges.

11. Composition selon l'une quelconque des revendications précédentes, dans laquelle l'huile présente une masse moléculaire supérieure ou égale à 250 g/mol, notamment entre 250 et 10 000 g/mol, de préférence supérieure ou égale à 300 g/mol, notamment entre 300 et 8000 g/mol et mieux, supérieure ou égale à 400 g/mol, notamment entre 400 et 5000 g/mol.

12. Composition selon l'une quelconque des revendications précédentes, dans laquelle, dans la phase grasse, le rapport de ou des huile(s) au(x) composé(s) particulier(s) est de 10/90 à 90/10, de préférence de 20/80 à 80/20 et de préférence encore de 30/70 à 70/30.

13. Composition selon l'une quelconque des revendications précédentes, qui est une composition anhydre.

14. Composition selon l'une quelconque des revendications 1 à 12, comprenant de l'eau ou un mélange d'eau et de solvant(s) organique (s) hydrophile (s).

15. Composition selon la revendication 8, dans laquelle le (s) solvant (s) organique (s) hydrophile (s) est (sont) choisi (s) parmi les monoalcools ayant de 2 à 5 atomes de carbone, les polyols ayant de 2 à 8 atomes de carbone, les cétones en C₃-C₄ et les aldéhydes en C₂-C₄.

16. Composition selon l'une quelconque des revendications 14 et 15, dans laquelle l'eau ou le mélange d'eau et de solvant(s) organique (s) hydrophile(s) est présent en une teneur allant de 0,1% à 90% en poids, par rapport au poids total de la composition, et de préférence de 0,1% à 60% en poids.

17. Composition selon l'une quelconque des revendications précédentes, **caractérisée en ce qu'**elle comprend un polymère filmogène.

18. Composition selon la revendication 17, dans laquelle le polymère filmogène est choisi dans le groupe formé par les polymères vinyliques, les polyuréthanes, les polyesters, les polyamides, les polyurées, et les polymères cellulosiques.

19. Composition selon la revendication 18, dans laquelle le polymère filmogène est présent en une teneur en matières sèches de polymère allant de 0,1% à 60% en poids par rapport au poids total de la composition, de préférence de 0,5% à 40% en poids, et mieux de 1% à 30% en poids.

20. Composition selon l'une quelconque des revendications précédentes, **caractérisée en ce qu'**elle comprend une matière colorante.

21. Composition selon la revendication 20, dans laquelle la matière colorante est choisie parmi les pigments, les nacres, les colorants liposolubles, et les colorants hydrosolubles.

22. Composition selon la revendication 20 ou la revendication 21, dans laquelle la matière colorante est présente en une teneur allant de 0,01% à 30% en poids, par rapport au poids total de la composition.

23. Composition selon l'une quelconque des revendications précédentes, **caractérisée en ce qu'**elle comprend un additif cosmétique choisi parmi les antioxydants, les charges, les conservateurs, les parfums, les neutralisants, les épaississants, les tensioactifs, les actifs cosmétiques ou dermatologiques, les agents plastifiants, les agents de coalescence et leurs mélanges.

24. Composition selon l'une quelconque des revendications précédentes, qui est une composition de maquillage, une base de maquillage, une composition dite « top-coat » à appliquer sur un maquillage, ou une composition de traitement, de soin, des fibres kératiniques.

25. Composition selon l'une quelconque des revendications précédentes qui est une composition de revêtement des cils, notamment une composition de maquillage des cils, encore appelé mascara, une composition à appliquer sur un maquillage des cils, dite encore top-coat, ou bien encore une composition de traitement des cils, notamment des cils d'êtres humains ou des faux cils.

26. Composition selon la revendication 25, qui est un mascara.

27. Procédé cosmétique de traitement ou de maquillage des fibres kératiniques comprenant l'application sur lesdites fibres kératiniques d'une composition selon l'une quelconque des revendications 1 à 26.

28. Procédé de revêtement des cils comprenant l'application sur les cils d'une composition selon l'une quelconque des revendications 1 à 26.

29. Utilisation d'une composition selon l'une quelconque des revendications 1 à 26 pour le maquillage des fibres kératiniques.

30. Utilisation d'une composition selon l'une quelconque des revendications 1 à 26, pour obtenir une application facile et homogène et un maquillage présentant un excellent effet volumateur et séparant.
